# EUROPÄISCHE PATENTSCHRIFT

(11) **EP 0 499 054 B1**
(45) Veröffentlichungstag und Bekanntmachung des Hinweises auf die Patenterteilung: **27.03.1996**
(21) Anmeldenummer: 92100957.7
(22) Anmeldetag: 22.01.1992
(51) Int. Cl.: C07D 303/04, C07D 301/19, C07C 45/33, C07C 49/403, C07C 29/50, C07C 35/08, C07C 409/14

(54) **Kreislaufverfahren zur Herstellung von Cyclohexenoxid sowie Cyclohexanol und Cyclohexanon**
Cyclic process for the preparation of cyclohexene oxide as well as of cyclohexanol and cyclohexanone
Procédé cyclique pour la préparation d'oxyde de cyclohexène ainsi que de cyclohexanol et de cyclohexanone

(30) Priorität: 14.02.1991 DE 4104419
(43) Veröffentlichungstag der Anmeldung: 19.08.1992
(73) Patentinhaber: BASF Aktiengesellschaft, D-67063 Ludwigshafen (DE)
(72) Erfinder: Agar, David, Dr., W-6149 Rimbach (DE); Bever, Paul-Michael, Dr., W-6800 Mannheim 1 (DE); Schuster, Hans H., Dr., W-6701 Erpolzheim (DE); Neubauer, Gerald, Dr., W-6940 Weinheim (DE)

(56) Entgegenhaltungen:
- EP-A- 0 268 826
- GB-A- 1 337 300
- WORLD PATENTS INDEX LATEST Week 8106, Derwent Publications Ltd., London, GB; AN 81-09287D

## Beschreibung

Aus der EP-A 92 867 ist bekannt, daß man Cyclohexylhydroperoxid enthaltende Reaktionsgemische aus der Cyclohexanoxidation mit wäßrigen Lösungen von Alkalimetallhydroxiden die ggf. katalytisch wirksame Metallsalze enthalten können, behandelt, um das im Reaktionsgemisch enthaltene Cyclohexylhydroperoxid in Cyclohexanol und Cyclohexanon umzuwandeln. Dieses Verfahren hat den Nachteil, daß man erhebliche Mengen an Alkalimetallhydroxiden anwenden muß, die zu entsorgen sind und zudem beträchtliche Mengen an Cyclohexanon und/oder Cyclohexanol unter Bildung von Hochsiedern verloren gehen.

In der DE-A 24 04 950 wird ein Verfahren zur Herstellung von Cyclohexenoxid durch Umsetzen von Cyclohexen mit Cyclohexylhydroperoxid in Gegenwart von löslichen Titankatalysatoren unter Mitverwendung von Phosphorsäureestern beschrieben. Es wird jedoch kein Hinweis gegeben, wie dieses Verfahren mit dem Verfahren zur Herstellung von Cyclohexylhydroperoxid verbunden werden soll.

Aus der DE-A 36 36 056 ist bereits zu entnehmen, daß man Cyclohexylhydroperoxid, das durch Oxidation von Cyclohexan erhalten worden ist, in der bei der Oxidation anfallenden Lösung mit Gemischen aus Cyclohexen und Cyclohexan in Gegenwart von löslichen Metallkatalysatoren der 4. bis 6. Nebengruppe des Periodischen Systems umsetzt und anschließend das in der Lösung enthaltene Cyclohexenoxid zu Cyclohexanol hydriert. Bemerkenswert ist, daß Cyclohexen im Überschuß, bezogen auf Cyclohexylhydroperoxid, verwendet wird, das ebenfalls hydriert wird. Es wird kein Hinweis gegeben, wie zu verfahren ist, um den Zwangsanfall an Cyclohexan zu mindern.

Es war deshalb die technische Aufgabe gestellt, ein Kreislaufverfahren zur Herstellung von Cyclohexenoxid, Cyclohexanol sowie Cyclohexanon zur Verfügung zu stellen, bei dem möglichst wenig nicht verwertbare Nebenprodukte anfallen, ein optimaler Verbund zwischen Cyclohexanoxidation und Epoxidierung von Cyclohexen erzielt wird und darüber hinaus die Aufwendungen für die Aufarbeitung minimiert werden.

Diese Aufgabe wird gelöst in einem Kreislaufverfahren zur Herstellung von Cyclohexenoxid sowie Cyclohexanol und Cyclohexanon, welches folgende Schritte umfaßt:
a) Herstellung eines im wesentlichen aus Cyclohexylhydroperoxid, Cyclohexanol, Cyclohexanon und Cyclohexan bestehenden Gemisches durch Oxidation von Cyclohexan mit molekularem Sauerstoff oder solches enthaltenden Gasen bei einer Temperatur von 130 bis 200°C und unter einem Druck von 5 bis 125 bar in flüssiger Phase
b) Gemeinsames Auftrennen des Gemisches aus Stufe a) und einem in Stufe c) anfallenden Cyclohexenoxid enthaltenden Epoxidierungsgemisches durch Destillation in
   b₁) eine im wesentlichen aus Cyclohexan und Cyclohexen bestehende Fraktion, die nach Hydrierung in die Stufe a) zurückgeführt wird
   b₂) eine im wesentlichen aus Cyclohexenoxid bestehende Fraktion
   b₃) ein im wesentlichen aus Cyclohexanol und Cyclohexanon bestehendes Gemisch und
   b₄) ein im wesentlichen aus Cyclohexylperoxid, Cyclohexanol und Cyclohexanon sowie Katalysator bestehende Fraktion
   und
c) Umsetzen des als Fraktion b₄) erhaltenen Cyclohexylhydroperoxid enthaltenden Gemisches mit Cyclohexen im Gemisch mit Cyclohexan bei erhöhter Temperatur in Gegenwart von Verbindungen von Übergangsmetallen der 4. bis 6. Gruppe des Periodischen Systems unter Erhalt eines Epoxidierungsgemisches, das im wesentlichen aus Cyclohexenoxid, Cyclohexanol, Cyclohexanon, Cyclohexen und Cyclohexan sowie Katalysator besteht und das in Stufe b) gemeinsam mit dem Reaktionsgemisch aus a) aufgetrennt wird.

Das neue Verfahren hat den Vorteil, daß sowohl die Herstellung von Cyclohexylhydroperoxid neben Cyclohexanol und Cyclohexanon als auch Cyclohexenoxid durch Umsetzung von Cyclohexen mit Cyclohexylhydroperoxid in einem kombinierten Kreislaufverfahren als auch die gemeinsame Aufarbeitung ermöglicht wird. Weiter hat das neue Verfahren den Vorteil, daß die verwendeten Katalysatoren im Kreis geführt werden und zudem die Bildung von nicht verwendbaren Nebenprodukten minimiert wird. Ferner hat das neue Verfahren den Vorteil, daß eine aufwendige Trennung von Cyclohexen und Cyclohexan entfällt. Zudem wird es ermöglicht, bis zu 2 Mol Cyclohexanol je Mol Cyclohexylhydroperoxid zu erzeugen.

In der Stufe a) wird ein im wesentliches aus Cyclohexylhydroperoxid Cyclohexanol, Cyclohexanon und Cyclohexan bestehendes Gemisch durch Oxidation von Cyclohexan mit molekularem Sauerstoff oder solches enthaltenden Gasen bei einer Temperatur von 130 bis 200°C und unter einem Druck von 5 bis 125 bar in flüssiger Phase hergestellt. Gegebenenfalls werden Katalysatoren wie Cobaltsalze mitverwendet. Vorteilhaft führt man die Oxidation bis zu einem einem Umsatz von 2 bis 8 Gew.-%, bezogen auf eingesetztes Cyclohexan, durch. Druck und Temperatur werden so aufeinander abgestimmt, daß zu jedem Zeitpunkt die Umsetzung in flüssiger Phase erfolgt.

Nach einem geeigneten Verfahren führt man die Oxidation von Cyclohexan z.B. in einer senkrecht stehenden Reaktionszone durch, in der Lochbleche in gleichmäßigen Abständen angeordnet sind. Die Lochbleche haben vorteilhaft einen freien Querschnitt von 3 bis 20 %. Oberhalb jeden Lochblechs sind über dem Querschnitt gleichmäßig verteilt Düsen angeordnet, durch die Sauerstoff enthaltende Gase insbesondere Luft zugeführt werden. Durch die Reaktionszone leitet man von unten nach oben Cyclohexan, gleichzeitig führt man molekularen Sauerstoff enthaltende Gase, z.B. mit einem Sauerstoffgehalt von 5 bis 30 Vol.-%, vorteilhaft Luft durch jede Düsenöffnung zu. Die Menge an zugeführten molekularen Sauerstoff enthaltenden Gasen und Cyclohexan wird so aufeinander abgestimmt, daß das aus der Reaktionszone austretende Abgas einen Gehalt an molekularem Sauerstoff von nicht mehr als 0,1 bis 1,5 Vol.-% hat. Ein geeignetes Verfahren wird beispielsweise beschrieben in der EP-B 135 718. Die erhaltene Lösung kann zweckmäßig vor der Weiterverarbeitung auch durch partielles Abdestillieren von Cyclohexan z.B. durch Entspannungsdestillation, an Cyclohexylhydroperoxid angereichert werden.

Typische Reaktionsgemische enthalten beispielsweise neben Cyclohexan 0,5 bis 5,0 Gew.-% Cyclohexylhydroperoxid 0,1 bis 2,5 Gew.-% Cyclohexanol sowie 0,1 bis 1,5 Gew.-% Cyclohexanon, ferner Nebenprodukte wie Ester und Carbonsäuren. Vorteilhaft werden solche Reaktionsgemische vor der Weiterbehandlung mit Wasser und/oder wäßrigen Alkalicarbonatlösungen gewaschen.

In der Stufe b) wird das Cyclohexylhydroperoxid enthaltende Gemisch aus Stufe a) und ein in der Stufe c) anfallendes Cyclohexenoxid enthaltendes Epoxidierungsgemisch, das im wesentlichen aus Cyclohexanol, Cyclohexanon, Cyclohexenoxid, Cyclohexen, Cyclohexan und Katalysator besteht, gemeinsam durch Destillation aufgetrennt. Vorteilhaft mischt man das zu destilierende Gemisch aus Stufe a) und Stufe c) vor Einleiten in die Kolonne. Bei der Destillation trennt man in folgende Fraktionen auf:
b₁) eine im wesentlichen aus Cyclohexan und Cyclohexen bestehende Fraktion. Ein typisches Gemisch enthält beispielsweise 0,1 bis 5 Gew.-% Cyclohexen neben Cyclohexan. Die Cyclohexen enthaltende Fraktion wird hydriert und das hierbei erhaltene Cyclohexan als Ausgangsverbindung für die Oxidation in die Stufe a) zurückgeführt. Die Hydrierung führt man in Gegenwart von Hydrierkatalysatoren, insbesondere Metallen der 8. Gruppe des Periodischen Systems, z.B. Cobalt, Nickel oder Edelmetallen wie Palladium oder Platin bei einer Temperatur von 100 bis 200°C unter erhöhtem Druck, z.B. 10 bis 130 bar zweckmäßig in flüssiger Phase durch.

Als Fraktion b₂) wird eine im wesentlichen aus Cyclohexenoxid bestehende Fraktion gewonnen. In dem Cyclohexenoxid sind geringe Mengen an Verunreinigungen wie Cyclohexan, Cyclohexen, Cyclohexanol, Cyclohexanon und Cyclohexylhydroperoxid enthalten.

Als Fraktion b₃) wird ein im wesentliches aus Cyclohexanol und Cyclohexanon bestehendes Gemisch abgetrennt. Ein typisches Gemisch enthält beispielsweise 50 bis 95 Gew.-% Cyclohexanol und 5 bis 50 Gew.-% Cyclohexanon und bis zu 2 Gew.-% Verunreinigungen.

Als weitere Fraktion b₄) trennt man ein im wesentliches aus Cyclohexylhydroperoxid Cyclohexanol und Cyclohexanon sowie Katalysator bestehendes Gemisch ab. Die Katalysatoren sind nachfolgend näher beschrieben. Ein typisches Gemisch enthält z.B. 10 bis 20 Gew.-% Cyclohexylhydroperoxid neben Cyclohexanol und Cyclohexanon sowie 0,01 bis 5 Gew.-% Katalysator berechnet als Metall, bezogen auf Cyclohexylhydroperoxid.

In der Stufe c) wird die Cyclohexylhydroperoxid enthaltende Fraktion b₄) mit Cyclohexen im Gemisch mit Cyclohexan in Berührung gebracht und Cyclohexylhydroperoxid mit Cyclohexen unter Bildung von Cyclohexenoxid und Cyclohexanol umgesetzt. Vorteilhaft wendet man Cyclohexylhydroperoxid im Überschuß bezogen auf Cyclohexen an. Vorteilhaft beträgt das Verhältnis Cyclohexen zu Cyclohexylhydroperoxid 0,7 bis 1. Die Umsetzung wird bei erhöhter Temperatur durchgeführt. Vorteilhaft hält man eine Temperatur von 70 bis 150°C, insbesondere von 90 bis 140°C ein. Die Umsetzung wird in flüssiger Phase durchgeführt. Ferner beträgt die Verweilzeit vorteilhaft 15 bis 120 Minuten, insbesondere 30 bis 100 Minuten. Der Druck wird so gewählt, daß das Reaktionsgemisch keine Gasphase ausbildet und stets in flüssiger Phase vorliegt.

Die Umsetzung wird in Gegenwart von Übergangsmetallverbindungen der 4. bis 6. Gruppe des Periodischen Systems durchgeführt. Vorteilhaft sind die verwendeten Verbindungen in Cyclohexan löslich. Geeignete Metalle sind beispielsweise Titan, Zirkon, Vanadium, Niob, Tantal, Chrom, Molybdän oder Wolfram. Geeignete Verbindungen sind beispielsweise Acetylacetonate oder Salze mit höheren Fettsäuren, z.B. mit 8 bis 18 Kohlenstoffatomen wie 2-Ethylhexansäure, Undecansäure, Stearinsäure, Palmitinsäure oder Naphthenate. Besonders bevorzugt sind Verbindungen von Molybdän, Titan, Vanadium und Wolfram. Besondere Bedeutung haben Molybdänverbindungen erlangt. Vorteilhaft wendet man je Mol Cyclohexylhydroperoxid 0,1 bis 2 mMol der Katalysatoren berechnet als Metall an.

Cyclohexen wird im Gemisch mit Cyclohexan verwandt. Ein typisches Gemisch enthält beispielsweise 10 bis 30 Gew.-% Cyclohexen neben Cyclohexan. Zur Ergänzung des Katalysators wird der Stufe c) mit dem Cyclohexen und Cyclohexan frischer Katalysator zweckmäßig in gelöster Form zugeführt.

Man erhält ein Epoxidierungsgemisch, das im wesentlichen aus Cyclohexenoxid, Cyclohexanol, Cyclohexanon, Cyclohexen sowie gelösten Katalysator und Cyclohexan besteht. Ein typisches Gemisch enthält beispielsweise 0,1 bis 1 Gew.-% Cyclohexen, 5 bis 20 Gew.-% Cyclohexanol, 1 bis 15 Gew.-% Cyclohexanon, 1 bis 10 Gew.-% Cyclohexylhydroperoxid sowie 50 bis 90 Gew.-% Cyclohexan. Das so erhaltene Gemisch wird in der Stufe b) gemeinsam mit dem Oxidationsgemisch aus der Stufe a) durch Destillation getrennt.

Die Destillation des Gemischs aus den Stufen a) und b) wird zweckmäßig in zwei Kolonnen durchgeführt. Das zu destillierende Gemisch wird beispielsweise im mittleren Teil der ersten Kolonne zugeführt und am Kopf der Kolonne ein Gemisch aus Cyclohexan und Cyclohexen entnommen, während im Sumpf der Kolonne die Cyclohexylhydroperoxid enthaltende Fraktion b₄) entnommen wird. Cyclohexenoxid (Fraktion b₂) und das Gemisch aus Cyclohexanol und Cyclohexanon (Fraktion b₃) werden in einer im Nebenschluß geschalteten Kolonne entnommen. Vorteilhaft achtet man darauf, daß im Sumpf der ersten Kolonne eine Temperatur von 110°C nicht überschritten wird. Die Destillation wird in der Regel unter vermindertem Druck, z.B. bei 50 bis 300 mbar durchgeführt. Beim Abtrennen des Cyclohexylhydroperoxids wird vorteilhaft eine Verweilzeit von weniger als 120 Minuten vorzugsweise von 10 bis 60 Minuten eingehalten.

Das so erhaltene Cyclohexenoxid läßt sich in Gegenwart von Edelmetallkatalysatoren, z.B. Palladium zu Cyclohexanol hydrieren, das dann zusammen mit dem Gemisch aus Cyclohexanol und Cyclohexanon zur Herstellung von Faservorprodukten verwendbar ist.

Das Verfahren nach der Erfindung sei an folgenden Beispielen veranschaulicht.

### Beispiel

Zur Epoxidation wurde ein Gemisch der folgenden Zusammensetzung eingesetzt:

| | | |
|---|---|---|
| A) | Cyclohexen: | 6,8 Gew.-% |
| | Cyclohexenoxid: | 0,03 Gew.-% |
| | Cyclohexanon: | 0,93 Gew.-% |
| | Cyclohexanol: | 1,97 Gew.-% |
| | Cyclohexylhydroperoxid: | 11,6 Gew.-% |
| Rest hier und im folgenden Cyclohexan. | | |

Die Lösung enthielt 0,5 mmol Mo/mol CHHP als Katalysator. Sie wurde in einem Reaktor bei 110°C und einer Verweilzeit von 60 min umgesetzt. Das Produktgemisch enthielt nach der Umsetzung:

| | | |
|---|---|---|
| B) | Cyclohexen: | 0,39 Gew.-% |
| | Cyclohexenoxid: | 5,78 Gew.-% |
| | Cyclohexanon: | 2,03 Gew.-% |
| | Cyclohexanol: | 9,35 Gew.-% |
| | Cyclohexylhydroperoxid: | 0,91 Gew.-% |

Dem so erhaltenen Gemisch B wurde Rohoxidat aus der Cyclohexanoxidation zugesetzt und dann in einem Dünnschichtverdampfer im Vakuum bei 90°C eingedampft. Das Sumpfprodukt hatte folgende Zusammensetzung:

| | | |
|---|---|---|
| C) | Cyclohexen: | 0,05 Gew.-% |
| | Cyclohexenoxid: | 2,02 Gew.-% |
| | Cyclohexanon: | 2,14 Gew.-% |
| | Cyclohexanol: | 8,06 Gew.-% |
| | Cyclohexylhydroperoxid: | 12,7 Gew.-% |

Diesem Sumpfprodukt wurde soviel Cyclohexen zugegeben, daß das Verhältnis von Cyclohexylhydroperoxid zu Cyclohexen etwa 1/0,7 mol/mol betrug, bevor es zum Epoxidationsreaktor zurückgeführt wurde. Das Ausgangsprodukt vor Epoxidation hatte folgende Zusammensetzung:

| | |
|---|---|
| Cyclohexen: | 4,69 Gew.-% |
| Cyclohexenoxid: | 1,68 Gew.-% |
| Cyclohexanon: | 1,96 Gew.-% |
| Cyclohexanol: | 7,87 Gew.-% |
| Cyclohexylhydroperoxid: | 10,4 Gew.-% |

Die Reaktionsbedingungen entsprechen denjenigen für A. Das erhaltene Produkt hatte die Zusammensetzung:

| | |
|---|---|
| Cyclohexen: | 0,62 Gew.-% |
| Cyclohexenoxid: | 5,45 Gew.-% |
| Cyclohexanon: | 3,44 Gew.-% |
| Cyclohexanol: | 13,2 Gew.-% |
| Cyclohexylhydroperoxid: | 2,08 Gew.-%. |

## Patentansprüche

1. Kreislaufverfahren zur Herstellung von Cyclohexenoxid sowie Cyclohexanol und Cyclohexanon, welches folgende Schritte umfaßt:
a) Herstellen eines im wesentlichen aus Cyclohexylhydroperoxid, Cyclohexanol, Cyclohexanon und Cyclohexan bestehenden Gemisches durch Oxidation von Cyclohexan mit molekularem Sauerstoff oder solches enthaltenden Gasen bei einer Temperatur von 130 bis 200°C und unter einem Druck von 5 bis 125 bar in flüssiger Phase
b) gemeinsames Auftrennen des Gemisches aus der Stufe a) und einem in Stufe c) anfallenden Cyclohexenoxid enthaltenden Epoxidierungsgemisches durch Destillation in folgende Fraktionen
b₁) eine im wesentlichen aus Cyclohexan und Cyclohexen bestehende Fraktion, die nach Hydrierung in die Stufe a) zurückgeführt wird
b₂) eine im wesentlichen aus Cyclohexenoxid bestehende Fraktion
b₃) ein im wesentlichen aus Cyclohexanol und Cyclohexanon bestehendes Gemisch und
b₄) ein im wesentlichen aus Cyclohexylhydroperoxid, Cyclohexanol und Cyclohexanon sowie Katalysator bestehendes Gemisch
und
c) Umsetzen des als Fraktion b₄) erhaltenen Cyclohexylhydroperoxid enthaltenden Gemisches mit Cyclohexen im Gemisch mit Cyclohexan bei erhöhter Temperatur in Gegenwart von Übergangsmetallverbindungen der 4. bis 6. Gruppe des Periodischen Systems unter Erhalt eines Epoxidierungsgemisches, das im wesentlichen aus Cyclohexenoxid, Cyclohexanol, Cyclohexanon, Cyclohexen und Cyclohexan sowie Katalysator besteht und das in Stufe b) gemeinsam mit dem Gemisch aus a) durch Destillation getrennt wird.

2. Verfahren nach Anspruch 1, dadurch gekennzeichnet, daß man im Cyclohexan lösliche Molybdänverbindungen verwendet.

3. Verfahren nach den Ansprüchen 1 und 2, dadurch gekennzeichnet, daß man beim Abtrennen des Cyclohexylperoxid enthaltentenden Gemisches eine Verweilzeit von nicht mehr als 120 Minuten einhält.

4. Verfahren nach den Ansprüchen 1 bis 3, dadurch gekennzeichnet, daß man ein Molverhältnis von Cyclohexen zu Cyclohexylhydroperoxid von 0,7 bis 1 einhält.

## Claims

1. A cyclic process for the preparation of cyclohexene oxide, cyclohexanol and cyclohexanone which includes the following steps:
a) preparation of a mixture essentially comprising cyclohexyl hydroperoxide, cyclohexanol, cyclohexanone and cyclohexane by oxidizing cyclohexane using molecular oxygen or a gas containing molecular oxygen at from 130 to 200°C and at from 5 to 125 bar in the liquid phase,
b) joint distillative separation of the mixture from step a) and an epoxidation mixture produced in step c) and containing cyclohexene oxide, to give the following fractions
b₁) a fraction which essentially comprises cyclohexane and cyclohexene and is recycled into step a) after hydrogenation,
b₂) a fraction which essentially comprises cyclohexene oxide,
b₃) a mixture which essentially comprises cyclohexanol and cyclohexanone, and
b₄) a mixture which essentially comprises cyclohexyl hydroperoxide, cyclohexanol, cyclohexanone and catalyst,
and
c) reaction of the mixture obtained as fraction b₄) and containing cyclohexyl hydroperoxide with cyclohexene in a mixture with cyclohexane at elevated temperature in the presence of transition-metal compounds from group 4 to 6 of the periodic table, to give an epoxidation mixture which essentially comprises cyclohexene oxide, cyclohexanol, cyclohexanone, cyclohexene, cyclohexane and catalyst and is separated by distillation in step b) together with the mixture from a).

2. A process as claimed in claim 1, wherein molybdenum compounds which are soluble in cyclohexane are used.

3. A process as claimed in claim 1 or 2, wherein the separation of the mixture contianing cyclohexyl peroxide is carried out at a maximum residence time of 120 minutes.

4. A process as claimed in claims 1 to 3, wherein a molar ratio between cyclohexene and cyclohexyl hydropoeroxide of from 0.7 to 1 is maintained.

## Revendications

1. Procédé en circuit fermé pour la préparation d'oxyde de cyclohexène ainsi que de cyclohexanol et de cyclohexanone, comprenant les étapes suivantes;
a) Préparation d'un mélange qui se compose essentiellement d'hydroperoxyde de cyclohexyle, de cyclohexanol, de cyclohexanone et de cyclohexane, par oxydation de cyclohexane avec de l'oxygène moléculaire ou des gaz contenant celui-ci, à une température de 130 à 200°C et sous une pression de 5 à 125 bar en phase liquide,
b) Séparation en commun du mélange issu de l'étape a) et d'un mélange d'époxydation contenant de l'oxyde de cyclohexène, formé dans l'étape c), par distillation en les fractions suivantes;
b₁) une fraction qui se compose essentiellement de cyclohexane et de cyclohexène et qui est renvoyée, après hydrogénation, dans l'étape a),
b₂) une fraction se composant essentiellement d'oxyde de cyclohexène,
b₃) un mélange se composant essentiellement de cyclohexanol et de cyclohexanone et
b₄) un mélange se composant essentiellement d'hydroperoxyde de cyclohexyle, de cyclohexanol et de cyclohexanone ainsi que de catalyseur,
et
c) Mise en réaction du mélange contenant de l'hydroperoxyde de cyclohexyle, obtenu en tant que fraction b₄), avec du cyclohexène en mélange avec du cyclohexane, à température élevée et en présence de composés de métaux de transition des groupes IV à VI de la classification périodique, pour obtenir un mélange d'époxydation qui se compose essentiellement d'oxyde de cyclohexène, de cyclohexanol, de cyclohexanone, de cyclohexène et de cyclohexane ainsi que de catalyseur et qui est séparé par distillation dans l'étape b) en commun avec le mélange issu de a).

2. Procédé selon la revendication 1, caractérisé en ce que l'on utilise des composés du molybdène solubles dans le cyclohexane.

3. Procédé selon la revendication 1 ou 2, caractérisé en ce que l'on respecte, lors de la séparation du mélange contenant du peroxyde de cyclohexyle, une durée de séjour non supérieure à 120 min.

4. Procédé selon l'une quelconque des revendications 1 à 3, caractérisé en ce que l'on respecte un rapport molaire du cyclohexène à l'hydroperoxyde de cyclohexyle de 0,7 à 1.
